# EUROPEAN PATENT APPLICATION

(11) **EP 1 023 899 A1**
(43) Date of publication of application: **02.08.2000**
(21) Application number: 98944263.7
(22) Date of filing: 28.09.1998
(51) Int. Cl.: A61K 31/505, A61K 31/52, A61K 9/08, A61K 47/38

(54) **AQUEOUS PREPARATION CONTAINING ANTIVIRAL AGENT HAVING PURINE OR PYRIMIDINE SKELETON**

(30) Priority: 26.09.1997 JP 27797697
(71) Applicant: Wakamoto Pharmaceutical Co., Ltd., Chuo-ku, Tokyo 103-8330 (JP)
(72) Inventor: SUZUKI, Hidekazu, Wakamoto Pharm. Co., Ltd.,, Chuo-ku, Tokyo 103-8330 (JP); OGAWA, Hiroyuki, Wakamoto Pharm. Co., Ltd., Chuo-ku, Tokyo 103-8330 (JP); TAKEUCHI, Masanobu, Wakamoto Pharm. Co., Ltd,, Chuo-ku, Tokyo 103-8330 (JP); SAITO, Yoshiaki, Wakamoto Pharm. Co., Ltd., Chuo-ku, Tokyo 103-8330 (JP)
(74) Representative: VOSSIUS & PARTNER
(86) International application number: JP9804333
(87) International publication number: WO9916447

(57) **Abstract**

An aqueous preparation which is used as a liquid preparation such as an eye drop or nasal drop for infectious diseases attributable to herpesvirus, etc., contains as the active ingredient an antiviral agent having a purine or pyrimidine skeleton, and is prevented from untergoing crystal precipitation in a physiologically acceptable pH range. The aqueous preparation contains a crystal precipitation inhibitor to keep the drug dissolved in the physiologically acceptable pH range and thus prevent crystal precipitation.

## Description

### Technical Field

The present invention relates to an aqueous pharmaceutical preparation, which contains an antiviral agent having a purine skeleton or a pyrimidine skeleton and which is prepared so as to have a physiologically acceptable pH value.

### Background Art

The antiviral agents, each having a purine skeleton or a pyrimidine skeleton, represented by Acyclovir and Idoxuridine are drugs effective as remedies for treating infectious diseases attributable to herpesviruses such as chickenpox, herpes encephalitis-meningitis and herpes simplex; and infectious diseases attributable to cytomegaloviruses; and acquired immunodeficiency syndrome (AIDS).

However, it has been known that these antiviral agents are hardly soluble in water and that they have a very low solubility in water, in particular, at the physiological pH region. It has also been known that, even if they are forcibly converted into aqueous pharmaceutical preparations, crystals of these drugs separate out from the preparations within a short period of time. For this reason, it has been quite difficult to dissolve these antiviral agents in water around the physiological pH region and to thus prepare aqueous pharmaceutical preparations, which can be stored over a long period of time.

On the other hand, there has been an increasing demand for the development of aqueous pharmaceutical preparations comprising these drugs such as an eye drop and a nasal drop. In other words, there has been desired for the development of aqueous pharmaceutical preparations, which are prepared by dissolving these antiviral agents in water around the physiologically acceptable pH and which can be stored over a long period of time.

As an attempt for breaking the foregoing situation, there has been proposed a method which comprises the step of adjusting the pH of an Acyclovir-containing dispersion to the range of from 10 to 13 in the presence of, for instance, a stabilizer such as a carboxylic acid or a chelating agent and a sulfite or an oxycarboxylic acid to thus dissolve Acyclovir (Japanese Un-Examined Patent Publication (hereunder referred to as "J.P. KOKAI") No. Hei 7-247216).

In addition, J.P. KOKAI No. Hei 8-268892 discloses the use of polyvinyl pyrrolidone as an auxiliary agent for solubilizing Acyclovir. This patent demonstrates that a 0.2% Acyclovir aqueous pharmaceutical preparation can be prepared through the use of polyvinyl pyrrolidone in a concentration of 10%. However, the solubility of Acyclovir near the physiological pH is 0.14% as disclosed in this patent and therefore, this technique would not permit any drastic improvement in the solubility of Acyclovir.

US Patent No. 5,472,954 discloses a method in which Acyclovir is converted into a complex using a cyclodextrin derivative to thus improve the solubility thereof in water.

The report of Andrew X. Chen et al. (Pharm. Res., 1994, 11(3), pp. 398∼401) discloses a method for dissolving adenine as a nucleoside derivative, which makes use of tryptophan or saccharin. However, this article does not disclose any specific description concerning the effect of tryptophan or saccharin on the solubility, in water, of antiviral agents each carrying a purine skeleton or a pyrimidine skeleton. Moreover, this article never discloses any description about N-acetyl tryptophan at all. In addition, the article does not include any description concerning the storage stability of antiviral agents after the solubilization thereof.

There has been proposed a method for dissolving, for instance, cephalosporin derivatives and pyridone carboxylic add derivatives using N-acetyl tryptophan (J.P. KOKAI No. Hei 8-143475). However, this patent does not disclose any description concerning the effect of N-acetyl tryptophan on the solubility, in water, of antiviral agents each carrying a purine skeleton or a pyrimidine skeleton. In addition, the patent does not include any description concerning the storage stability of antiviral agents after the solubilization thereof.

### Disclosure of the Invention

The present invention relates to an aqueous pharmaceutical preparation, which comprises, as an effective component, an antiviral agent having a purine skeleton or a pyrimidine skeleton represented by Acyclovir or Idoxuridine and which comprises an agent for inhibiting crystal-separation. More specifically, the present invention pertains to an aqueous pharmaceutical preparation, which comprises an antiviral agent, N-acetyl tryptophan and/or saccharin and a pharmaceutically acceptable salt thereof and a water-soluble polymer as an optional auxiliary agent for inhibiting crystal-separation and whose pH is adjusted to a level near the physiologically acceptable value.

In the aqueous pharmaceutical preparation according to the present invention, an antiviral agent having a purine skeleton or a pyrimidine skeleton is dissolved in water at the physiologically acceptable pH region and any separation of crystals of the drug is inhibited during the storage of the preparation.

The aqueous pharmaceutical preparation of the present invention can be used as a remedy for treating, for instance, infectious diseases attributable to herpesviruses such as chickenpox, herpes encephalitis-meningitis and herpes simplex; and infectious diseases attributable to cytomegaloviruses; and acquired immunodeficiency syndrome (AIDS), in the form of an eye drop, a nasal drop, an ear drop, an inhalant, an aerosol, a liquid preparation for internal use and an injection.

### Best Mode for Carrying out the Invention

In the present invention, the term "aqueous pharmaceutical preparation" means a pharmaceutical preparation in the form of an aqueous solution, which comprises a drug dissolved therein and which is clear or transparent.

The antiviral agent having a purine skeleton or a pyrimidine skeleton used in the present invention is not restricted to any specific one, but preferred are those listed below: Acyclovir (2-amino-1,9-dihydro-9-((2-hydroxyethoxy) methyl)-6H-purin-6-one), Gancyclovir (2-amino-1,9-dihydro-9-((2-hydroxy-1-(hydroxymethyl) ethoxy) methyl)-6H-purin-6-one), Famciclovir (2-(2-(2-amino-9H-purin-9-yl)ethyl)-1,3-propanediol diacetate ester), Valacyclovir (L-valine 2-((2-amino-1,6-dihydro-6-oxo-9H-purin-9-yl) methoxy)ethyl ester), Penciclovir (2-amino-1,9-dihydro-9-(4-hydroxy-3-(hydroxymethyl)butyl)-6H-purin-6-one), Zanamivir (5-(acetylamino)-4-((aminoiminomethyl) amino)-2,6-anhydro-3,4,5-trideoxy-D-glycero-D-galacto-non-2-enonic acid), Adefovir dipivoxil (2,2-dimethyl-propanoic acid (((2-(6-amino-9H-purin-9-yl) ethoxy) methyl)-phosphinylidene) bis(oxymethylene) ester), Lobucavir (2-amino-9-((1R,2R,3S)-2,3-bis(hydroxymethyl)-cyclobutyl)-1,9-dihydro-6H-purin-6-one), Didanosine (2',3'-dideoxy-inosine), Vidarabine (9-β-D-arabinofuranosyl-9H-purin-6-amine), Idoxuridine (2'-deoxy-5-iodo-uridine), Trifluorothymidine (α,α,α - trifluoro-thymidine), cytosine arabinoside (4-amino-1-β-D-arabinofuranosyl-2(1H)-pyrimidinone), bromodeoxyuridine (5-bromo-2'-deoxyuridine), 5-iodo-2-deoxycytidine, Solibudine (1-β-D-arabinofuranosyl-5-((1E)-2-bromoethenyl)-2,4-(1H,3H)-pyrimidine-dione), Cidofovir ((((1S)-2-(4-amino-2-oxo-1-(2H)-pyrimidinyl)-1-(hydroxymethyl) ethoxy) methyl)-phosphonic acid) and pharmaceutically acceptable salts thereof, with Acyclovir, Gancyclovir, 'Vidarabine, Idoxuridine, Trifluorothymidine, cytosine arabinoside, bromodeoxyuridine, 5-iodo-2-deoxycytidine and pharmaceutically acceptable salts thereof being particularly preferred. These pharmaceutically acceptable salts may be, for instance, hydrochlorides, sulfates, nitrates, acetates, citrates, sodium salts, potassium salts and calcium salts.

The concentration of the antiviral agent having a purine skeleton or a pyrimidine skeleton used in the present invention ranges from 0.01 to 3 W/V% and preferably 0.1 to 0.9 W/V%. The term "W/V%" herein used will hereinafter be referred to as simply "%" unless otherwise specified.

This is because, if the concentration of the antiviral agent is less than 0.01%, any sufficient therapeutic effect cannot be expected, while if the concentration exceeds 3%, the drug is not completely solubilized in water and accordingly, any satisfactory aqueous pharmaceutical preparation cannot be prepared.

The agent for inhibiting crystal-separation may be phthalic acid, N-acetyl tryptophan, saccharin or pharmaceutically acceptable salts thereof, but preferred are N-acetyl tryptophan, saccharin or a pharmaceutically acceptable salt thereof.

N-Acetyl tryptophan may be D-, L- and DL-optical isomers or may be pharmaceutically acceptable salts thereof. The pharmaceutically acceptable salts may be, for instance, sodium salts, potassium salts and calcium salts.

In addition, saccharin usable herein may likewise be pharmaceutically acceptable salts thereof such as sodium salts, potassium salts and calcium salts.

Saccharin can easily be available from Daito Chemical Co., Ltd. (Daito-kagaku), Daiwa Chemical Industry Co., Ltd. (Daiwa-kasei) and Taiyo Chemical Industry Co., Ltd. (Taiyo-kagakukogyo) and sodium salt of saccharin may be available from Fuji Amide Chemical Co., Ltd., Daito Chemical Co., Ltd., Daiwa Chemical Industry Co., Ltd., Taiyo Chemical Industry Co., Ltd. and Tanabe Seiyaku. Co., Ltd.

These N-acetyl tryptophan, saccharin and pharmaceutically acceptable salts thereof may be used alone, but are preferably used in combination because of the synergistic effect thereof.

The concentration of N-acetyl tryptophan or a pharmaceutically acceptable salt thereof to be used in general ranges from 0.1 to 35%, preferably 1 to 20% and more preferably 1 to 10%. The concentration of saccharin or a pharmaceutically acceptable salt thereof to be used in general ranges from 0.1 to 68%, preferably 1 to 20% and more preferably 1 to 10%. This is because if the concentration of N-acetyl tryptophan or saccharin is less than 0.1%, the drug is not completely solubilized and thus cannot be converted into an aqueous pharmaceutical preparation. On the other hand, the concentration of N-acetyl tryptophan exceeds 35% or that of saccharin exceeds 68%, these agents for inhibiting crystal-separation cannot completely be dissolved in water.

The foregoing agent for inhibiting crystal-separation is preferably used in combination with a water-soluble polymer as an auxiliary agent for inhibiting crystal-separation, because of their synergistic effect. The water-soluble polymer is not restricted to any particular one, but preferred are, for instance, methyl cellulose, hydroxypropylmethyl cellulose, polyvinyl alcohol, polyethylene glycol, chondroitin sulfate, hyaluronic acid, alginic acid, polyvinyl pyrrolidone, carboxyvinyl polymer, chitosan and pharmaceutically acceptable salts thereof.

Examples of pharmaceutically acceptable salts of chondroitin sulfate, hyaluronic acid, alginic acid and carboxyvinyl polymer may include sodium salts, potassium salts and calcium salts. Pharmaceutically acceptable salts of chitosan may be, for instance, hydrochloride and sulfate.

Methyl cellulose may easily be commercially available from, for instance, Shin-Etsu Chemical Co., Ltd. under the trade name of Metolose (registered trade mark) SM, Dow Chemical Japan Ltd. under the trade name of Metocel A and Matsumoto Fats & Oils Co., Ltd. (Matsumotoyushiseiyaku) under the trade name of Mapolose M.

Hydroxypropylmethyl cellulose may easily be commercially available from Shin-Etsu Chemical Co., Ltd. under the trade name of Metolose (registered trade mark) 90SH, 65SH, 60SH and TC-5, Dow Chemical Japan Ltd. under the trade name of Metocel K, F and E and Matsumoto Fats & Oils Co., Ltd. under the trade name of Mapolose.

Polyvinyl alcohol may be easily commercially available from The Nippon Synthetic Chemical Industry Co., Ltd. under the trade name of Gosenol, Shin-Etsu Chemical Co., Ltd. under the trade name of Shin-Etsu Poval, Denki Kagaku Kogyo K.K. under the trade name of DENKA PVA, Kuraray Co., Ltd. under the trade name of KURARAY PVA and Unitika Ltd. under the trade name of UNITIKA Poval.

Polyethylene glycol may easily be commercially available from Nippon Soda Co., Ltd. under the trade name of NISO Polyethylene Glycol #200, 300, 400, 600, 1000, 1500, 1540, 4000, 6000 and 20000; and Lion Corporation, Daiichi Seiyaku Kogyo Co., Ltd., Asahi Denka Kogyo K.K., Sanyo Chemical Industries, Ltd. and NOF Corporation, under the trade name of Macrogol 200, 300, 400, 600, 1000, 1500, 1540, 4000, 6000 and 20000. Moreover, Macrogol may likewise easily be commercially available from Toho Chemical Industry Co., Ltd. under the trade name of Macrogol 200; and Maruishi Seiyaku K.K. under the trade name of Macrogol 400 and 4000.

Chondroitin sulfate may easily be commercially available from Kaken Pharmaceutical Co., Ltd. and Seikagaku Corporation in the form of sodium chondroitin sulfate. Hyaluronic acid may easily be commercially available from Iwase Cospha K.K., Nikko Chemicals Co., Ltd., Chisso Corporation, and Kimura Sangyo K.K. in the form of sodium hyaluronate. Alginic acid may easily be commercially available from Kimitsu Chemical Industry Co., Ltd. (Kimitsukagaku-kogyo), Fuji Chemical Industry Co., Ltd. (Fujikagaku-kogyo), and Saneigen F.F.I. K.K. in the form of alginic acid. In addition, sodium alginate may easily be commercially available from Kimitsu Chemical Industry Co., Ltd. under the trade name of KIMITSU Algin; Kibun Food Chemifa Co., Ltd. under the trade name of DACK Algin; and Henkel Hakusui Co., Ltd. and Ajinomoto Co., Inc. under the trade name of Texamide.

Polyvinyl pyrrolidone may be Polyvinyl Pyrrolidone K25, K30, K90, which may easily be commercially available from, for instance, BASF Japan Co., Ltd. under the trade name of Kollidon (registered trade mark); and ISP Co., Ltd. and Gokyo Sangyo K.K. under the trade name of Purasudon. Carboxyvinyl polymer may easily be commercially available from BF Goodrich/Chugai Boeki K.K. under the trade name of Carbopol; Japan Pure Chemicals Co., Ltd. (Nippon-junyaku) under the trade name of Junlon; and Wako Pure Chemical Industries Co., Ltd. (Wakojunyaku-kogyo) under the trade name of HIVIS WAKO. Among these, particularly preferred are methyl cellulose and hydroxypropylmethyl cellulose.

The concentration of these polymers to be used is not restricted to any particular range, but the polymers are preferably used in such a concentration that the resulting aqueous pharmaceutical preparation has a viscosity ranging from about 2 to 1000 mPa·s, more preferably about 2 to 300 mPa·s and particularly preferably about 5 to 50 mPa·s. For instance, the concentration desirably ranges from about 0.2 to 3% when using Metolose (registered trade mark) SM-15 available from Shin-Etsu Chemical Co., Ltd. as methyl cellulose; about 0.5 to 2% when using Metolose (registered trade mark) 60SH 50 available from Shin-Etsu Chemical Co., Ltd. as hydroxypropylmethyl cellulose; about 1 to 5% when using Poly(vinyl alcohol) 1000 (Partially Hydrolyzed) as polyvinyl alcohol; and about 1.5 to 5% when using sodium chondroitin sulfate available from Seikagaku Corporation.

The pH value of the aqueous pharmaceutical preparation of the present invention ranges from 3 to 10 and preferably 5.5 to 8.0 in which the preparation never has an irritating action for mucous membranes and is not corrosive.

The aqueous pharmaceutical preparation of the present invention may if necessary comprise, for instance, a buffering agent, a preservative, a stabilizer and/or an isotonicity.

The foregoing buffering agent is not restricted to any particular one so far as it can generally and pharmaceutically be used and may be, for instance, acetic acid, phosphoric acid, boric acid, citric acid, tartaric acid, lactic acid, carbonic acid, amino acids and pharmaceutically acceptable salts thereof. Examples of amino acids include aspartic acid, arginine, glycine, and glutamic acid.

The foregoing preservative may be, for instance, quaternary ammonium salts such as benzalkonium chloride, benzethonium chloride and chlorhexidine gluconate; alcohols such as chlorobutanol, propylene glycol and benzyl alcohol; phenolic substances such as phenol, cresol, p-oxybenzoic acid esters; acidic substances such as benzoic acid, dehydroacetic acid, sorbic acid and sulfurous acid or pharmaceutically acceptable salts thereof.

As the foregoing stabilizers, there may be listed, for instance, antioxidants such as sodium nitrite, sodium sulfite, tocopherol acetate and ascorbic acid; and chelating agents such as ethylenediaminetetraacetic acid, citric acid and metal salts thereof.

Examples of the foregoing isotonicities are sodium chloride, potassium chloride, boric acid, borax, glycerin, propylene glycol, glucose, xylitol and mannitol.

In the method of the present invention, any known preparation method can be adopted, but the method of the present invention can be carried out as follows. For instance, methyl cellulose is dispersed in hot water, then cooled and N-acetyl tryptophan, saccharin and Acyclovir are added to the dispersion at an instance when the dispersion is converted into a clear solution, followed by mixing these components till the mixture becomes uniform. Then, sodium hydroxide is added to the system and the stirring is continued till all of the components are completely solubilized. If necessary, the mixture may be heated up to about 40°C. Hydrochloric acid is gradually added to this liquid in small portions to thus adjust the pH thereof to a desired level and the volume of the liquid is adjusted to a desired level by addition of water. Various additives such as a buffering agent, an isotonicity, a salt and a preservative may if necessary be added to the liquid.

The aqueous pharmaceutical preparation of the present invention may be sterilized by, for instance, filtration through a membrane or intermittent method.

Moreover, the aqueous pharmaceutical preparation of the invention may be charged in an eye drop bottle made of a plastic to thus use it as an eye drop. To store the same as an eye drop over a long period of time, the eye drop bottle can be packaged in a laminate bag comprising a polyethylene film and an aluminum foil together with an antioxidant (such as Ageless (registered trade mark) available from Mitsubishi Gas Chemical Co., Inc.) according to the pillow type packaging technique.

The aqueous pharmaceutical preparation can be charged in a dropping bottle made of a plastic material and used as an ear drop. To store the same as an ear drop over a long period of time, the dropping bottle can be packaged in a laminate bag, which comprises a polyethylene film and an aluminum foil, together with an antioxidant (such as Ageless (registered trade mark) available from Mitsubishi Gas Chemical Co., Inc.) according to the pillow type packaging technique.

The aqueous pharmaceutical preparation may be packed in a nasal volume regulating spray and may be used as a nasal drop. To store the same as a nasal drop over a long period of time, the nasal drop can be packaged in a laminate bag comprising a polyethylene film and an aluminum foil together with an antioxidant (such as Ageless (registered trade mark) available from Mitsubishi Gas Chemical Co., Inc.) according to the pillow type packaging technique.

The aqueous pharmaceutical preparation is filled in an ampule, then sealed by fusing the ampule and thus can be used as an injection (such as intravenous, intra-arterial, subcutaneous, intra-cutaneous, intramuscular, intramyeloporous, intraperitoneal, intraocular injections), a liquid preparation for internal use, an inhalant, and an aerosol. The preparation is charged in an appropriate container depending on the applications, for instance, a prescription bottle in case of a liquid preparation for internal use; an electric nebulizer in case of an inhalant; and an atomizer in case of an aerosol.

### Examples

The present invention will hereinafter be described in more detail with reference to the following Examples, but the present invention is not restricted to these specific Examples at all.

### Example 1

To a mixture of N-acetyl-L-tryptophan, saccharin and Acyclovir, there was added distilled water and they were mixed till a uniform dispersion was obtained. Then a 0.1N sodium hydroxide solution was added to the mixture with stirring till all of the components were completely dissolved. A 0.1N hydrochloric acid solution was gradually added to the resulting solution in small portions to thus adjust the pH value thereof to 8.0 and distilled water was added thereto to give 100 ml of a solution.

### Example 2

Methyl cellulose (Metolose (registered trade mark) SM-15: available from Shin-Etsu Chemical Co., Ltd.) was dispersed in hot water and then the resulting dispersion was cooled. Thereafter N-acetyl-L-tryptophan and Acyclovir were added to the dispersion at an instance when the dispersion became clear and the mixture was sufficiently stirred till a uniform dispersion was obtained. Then a 0.1N sodium hydroxide solution was added to the dispersion with stirring till all of the components were completely dissolved. A 0.1N hydrochloric acid solution was gradually added to the resulting solution in small portions to thus adjust the pH value thereof to 8.0 and distilled water was added thereto to give 100 ml of a solution.

### Example 3

Methyl cellulose (Metolose (registered trade mark) SM-15: available from Shin-Etsu Chemical Co., Ltd.) was dispersed in hot water and then the resulting dispersion was cooled. Thereafter saccharin and Acyclovir were added to the dispersion at an instance when the dispersion became clear and the mixture was sufficiently stirred till a uniform dispersion was obtained. Then a 0.1N sodium hydroxide solution was added to the dispersion with stirring till all of the components were completely dissolved. A 0.1N hydrochloric acid solution was gradually added to the resulting solution in small portions to thus adjust the pH value thereof to 8.0 and distilled water was added thereto to give 100 ml of a solution.

### Example 4

Distilled water was added to a mixture of N-acetyl-L-tryptophan and Acyclovir, followed by sufficient stirring of the mixture to give a uniform dispersion. Then a 0.1N sodium hydroxide solution was added to the dispersion with stirring till all of the components were completely dissolved. A 0.1N hydrochloric acid solution was gradually added to the resulting solution in small portions to thus adjust the pH value thereof to 8.0 and distilled water was added thereto to give 100 ml of a solution.

### Example 5

Distilled water was added to a mixture of saccharin and Acyclovir, followed by sufficient stirring of the mixture to give a uniform dispersion. Then a 0.1N sodium hydroxide solution was added to the dispersion with stirring till all of the components were completely dissolved. A 0.1N hydrochloric acid solution was gradually added to the resulting solution in small portions to thus adjust the pH value thereof to 8.0 and distilled water was added thereto to give 100 ml of a solution.

### Comparative Example 1

To Acyclovir, there was added distilled water while heating the mixture to about 40°C and the mixture was sufficiently mixed till a uniform dispersion was obtained. Then a 0.1N sodium hydroxide solution was added to the dispersion with stirring till Acyclovir was completely dissolved. A 0.1N hydrochloric acid solution was gradually added to the resulting solution in small portions to thus adjust the pH value thereof to 8.0 and distilled water was added thereto to give 100 ml of a solution.

### Comparative Example 2

Methyl cellulose (Metolose (registered trade mark) SM-15: available from Shin-Etsu Chemical Co., Ltd.) was dispersed in hot water and then the resulting dispersion was cooled. Thereafter Acyclovir was added to the dispersion at an instance when the dispersion became clear and the mixture was sufficiently stirred till a uniform dispersion was obtained. Then a 0.1N sodium hydroxide solution was added to the dispersion with stirring, while heating the dispersion to about 40°C till Acyclovir was completely dissolved. A 0.1N hydrochloric acid solution was gradually added to the resulting solution in small portions to thus adjust the pH value thereof to 8.0 and distilled water was added thereto to give 100 ml of a solution.

Each of the Acyclovir-containing aqueous pharmaceutical preparations prepared above was charged in a 5 ml volume ampule and then the ampule was sealed through fusion. Then these ampules were stored at 7, 25 or 40°C for about one month to evaluate the stability thereof. The stability was evaluated by determining whether crystals of Acyclovir were separated out from the content of the ampules after the storage or not. The results thus obtained are summarized in the following Table 1.

**Table 1**

| Ex. No. | pH | MC (%) | NAT (%) | SC (%) | ACV (%) | 7°C | | 25°C | 40°C |
|---|---|---|---|---|---|---|---|---|---|
| | | | | | | 3 days | 5 days | 4 weeks | 4 weeks |
| 1 | 8.0 | -- | 2.5 | 3 | 0.3 | ○ | ○ | ○ | ○ |
| 2 | 8.0 | 2 | 2.5 | -- | 0.3 | ○ | ○ | ○ | ○ |
| 3 | 8.0 | 2 | -- | 3 | 0.3 | ○ | ○ | ○ | ○ |
| 4 | 8.0 | -- | 5 | -- | 0.3 | ○ | X | ○ | ○ |
| 5 | 8.0 | -- | -- | 6 | 0.3 | ○ | X | ○ | ○ |
| 1* | 8.0 | -- | -- | -- | 0.3 | X | X | X | △ |
| 2* | 8.0 | 4 | -- | -- | 0.3 | X | X | X | △ |

| | | | | | | | | | |
|---|---|---|---|---|---|---|---|---|---|
| *: Comparative Example. | | | | | | | | | |
| ACV: Acyclovir; MC: methyl cellulose; NAT: N-acetyl tryptophan; SC: saccharin ○ : clear solution; X: separation of crystals; △: fine contaminant | | | | | | | | | |

As will be clear from the data listed in Table 1, any separation of Acyclovir crystals was inhibited in the formulations prepared in Examples 4 and 5 with N-acetyl tryptophan or saccharin incorporated therein, as compared with the Acyclovir aqueous solution free of any agent for inhibiting crystal-separation prepared in Comparative Example 1. This clearly indicates that the addition of an agent for inhibiting crystal-separation is quite effective. Moreover, As will be clear from the results observed for the formulation prepared in Comparative Example 2, the use of methyl cellulose alone never shows any effect of inhibiting crystal-separation and thus crystals were separated out from the formulation. In the formulations prepared in Examples 1, 2 and 3 wherein N-acetyl tryptophan, saccharin and/or methyl cellulose were used in combination (amount of each component was 1/2 time the amount of the same used alone), the effect of inhibiting crystal-separation was excellent as compared with the formulations prepared in Examples 4 and 5 and Comparative Example 2, wherein the foregoing agents for inhibiting crystal-separation were used separately. This clearly shows that a synergistic effect can be obtained by the use of N-acetyl tryptophan, saccharin and/or methyl cellulose in combination.

### Examples 6 to 7

Distilled water was added to a mixture of Acyclovir and N-acetyl-L-tryptophan or saccharin, followed by sufficient mixing to give a uniform dispersion. Then a 0.1N sodium hydroxide solution was added to the dispersion with stirring till all of the components were completely dissolved. A 0.1N hydrochloric acid solution was gradually added to the resulting solution in small portions to thus adjust the pH value thereof to 8.0 and distilled water was added thereto to give 100 ml of a solution.

### Examples 8 to 16

Methyl cellulose (Metolose (registered trade mark) SM-15: available from Shin-Etsu Chemical Co., Ltd.) was dispersed in hot water and then the resulting dispersion was cooled. Thereafter N-acetyl-L-tryptophan and/or saccharin and Acyclovir were added to the dispersion at an instance when the dispersion became clear and the mixture was sufficiently mixed till a uniform dispersion was obtained. Then a 0.1N sodium hydroxide solution was added to the dispersion with stirring till all of the components were completely dissolved. A 0.1N hydrochloric acid solution was gradually added to the resulting solution in small portions to thus adjust the pH value thereof to 8.0 and distilled water was added thereto to a total volume of the solution of 100 ml.

### Example 17

Methyl cellulose (Metolose (registered trade mark) SM-15: available from Shin-Etsu Chemical Co., Ltd.) was dispersed in hot water and then the resulting dispersion was cooled. Thereafter N-acetyl-L-tryptophan, saccharin and Acyclovir were added to the dispersion at an instance when the dispersion became clear and the mixture was sufficiently mixed till a uniform dispersion was obtained. Then a 0.1N sodium hydroxide solution was added to the dispersion with stirring till all of the components were completely dissolved. A 0.1N hydrochloric acid solution was gradually added to the resulting solution in small portions to thus adjust the pH value thereof to 5.5 and distilled water was added thereto to a total volume of the solution of 100 ml.

### Example 18

Methyl cellulose (Metolose (registered trade mark) SM-15: available from Shin-Etsu Chemical Co., Ltd.) was dispersed in hot water and then the resulting dispersion was cooled. Thereafter N-acetyl-DL-tryptophan, saccharin and Acyclovir were added to the dispersion at an instance when the dispersion became clear and the mixture was sufficiently mixed till a uniform dispersion was obtained. Then a 0.1N sodium hydroxide solution was added to the dispersion with stirring till all of the components were completely dissolved. A 0.1N hydrochloric acid solution was gradually added to the resulting solution in small portions to thus adjust the pH value thereof to 8.0 and distilled water was added thereto to a total volume of the solution of 100 ml.

### Comparative Example 3

Distilled water was added to Acyclovir and a 0.1N sodium hydroxide solution was added to the mixture with stirring under heating to 40°C till Acyclovir was completely dissolved. To the resulting liquid, there was added L-tryptophan dissolved in a 0.1N hydrochloric acid solution and further a 0.1N hydrochloric acid solution was gradually added to the resulting solution in small portions to thus adjust the pH value thereof to 8.0 and distilled water was added thereto to a total volume of the solution of 100 ml.

Each of the Acyclovir-containing aqueous pharmaceutical preparations prepared above was charged in a 5 ml volume ampule and then the ampule was sealed through fusion. Then these ampules were stored at 7, 25 or 40°C for about one month to evaluate the stability thereof. The stability was evaluated by determining whether crystals of Acyclovir were separated out from the content of the ampules after the storage or not. The results thus obtained are summarized in the following Table 2.

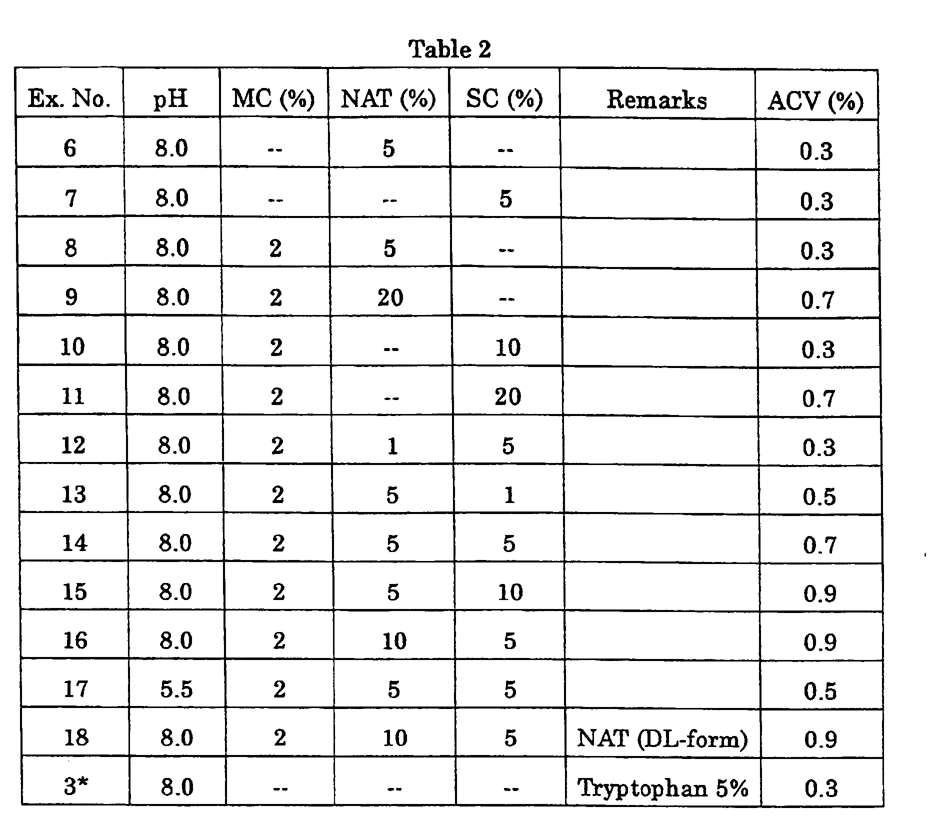

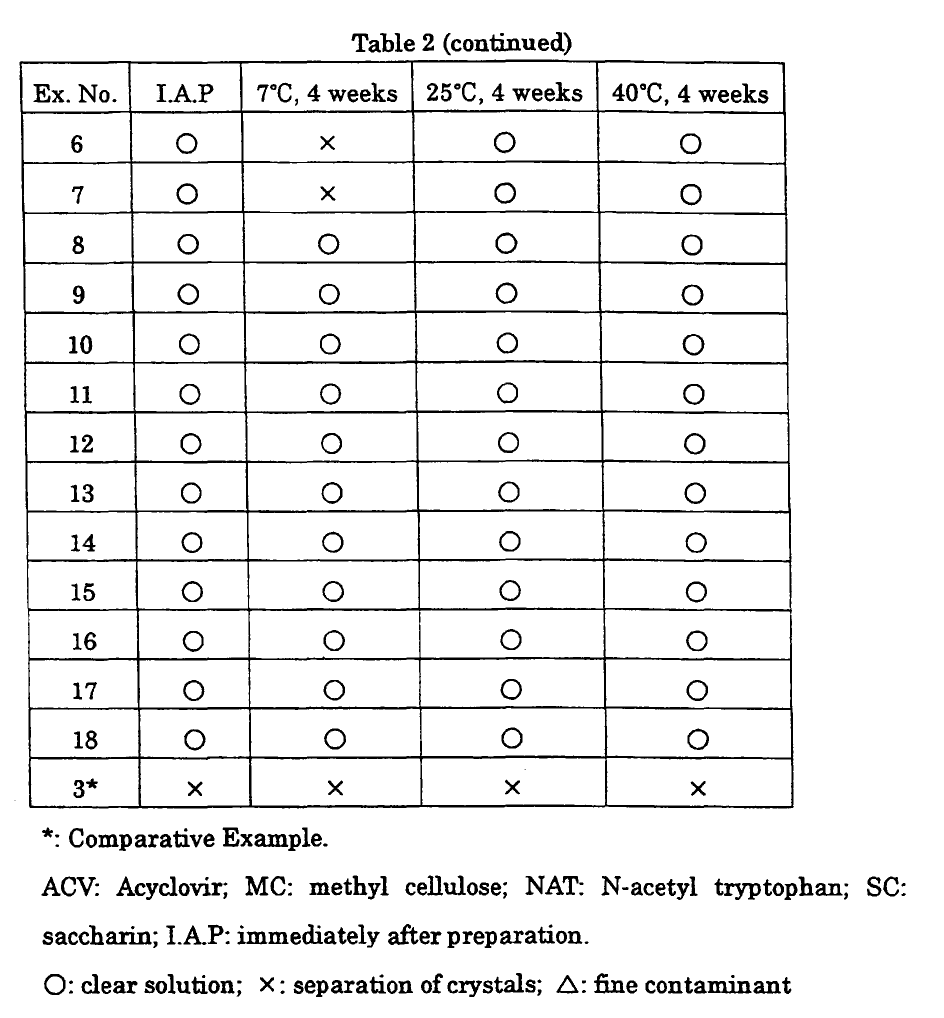

In Table 2, Example 6 corresponds to an example in which N-acetyl tryptophan is used as an agent for inhibiting crystal-separation. Contrary to this, Comparative Example 3 corresponds to an example in which tryptophan was substituted for N-acetyl tryptophan. However, it was found that Acyclovir or tryptophan was not dissolved in water and therefore, any aqueous pharmaceutical preparation could not be prepared.

In addition, the results of Example 8 clearly indicate that the effect of inhibiting crystal-separation can further be improved by the addition of methyl cellulose to the formulation prepared in Example 6. Moreover, the results of Examples 9 and 11 indicate that the use of the agent for inhibiting crystal-separation in an amount of 20% can inhibit the separation of any crystal of Acyclovir even when the amount of Acyclovir is 0.7%.

In addition, the results of Example 17 indicate that the aqueous pharmaceutical preparation of the present invention can be stored even at a pH value of 5.5.

Further the results observed for the formulations prepared in Examples 16 and 18 indicate that N-acetyl tryptophan can ensure the desired effect irrespective of the kinds of optical isomers thereof.

### Example 19

Distilled water was added to a mixture of N-acetyl-L-tryptophan, saccharin and Acyclovir and they were sufficiently mixed together to give a uniform dispersion. Then a 0.1N sodium hydroxide solution was added to the dispersion with stirring till all of the components were completely dissolved. A 0.1N hydrochloric acid solution was gradually added to the resulting solution in small portions to thus adjust the pH value thereof to 8.0 and distilled water was added thereto to a total volume of the solution of 100 ml.

### Example 20

Methyl cellulose (Metolose (registered trade mark) SM-15: available from Shin-Etsu Chemical Co., Ltd.) was dispersed in hot water and then the resulting dispersion was cooled. Thereafter N-acetyl-L-tryptophan, saccharin and Acyclovir were added to the dispersion at an instance when the dispersion became clear and they were sufficiently mixed till a uniform dispersion was obtained. Then a 0.1N sodium hydroxide solution was added to the dispersion with stirring till all of the components were completely dissolved. A 0.1N hydrochloric acid solution was gradually added to the resulting solution in small portions to thus adjust the pH value thereof to 8.0 and distilled water was added thereto to a total volume of the solution of 100 ml.

### Example 21

Hydroxypropylmethyl cellulose (Metolose (registered trade mark) 60SH 50, available from Shin-Etsu Chemical Co., Ltd.) was dispersed in hot water and the resulting dispersion was cooled. Then N-acetyl-L-tryptophan, saccharin and Acyclovir were added to the dispersion and they were sufficiently mixed together till a uniform dispersion was obtained. Then a 0.1N sodium hydroxide solution was added to the dispersion with stirring till all of the components were completely dissolved. A 0.1N hydrochloric acid solution was added to the resulting solution in small portions to adjust the pH value thereof to 8.0 and distilled water was added thereto to a final volume of the solution of 100 ml.

### Example 22

Polyvinyl alcohol (Poly(vinyl alcohol) 1000, available from Wako Pure Chemical Industry Co., Ltd.) was dissolved in hot water, then N-acetyl-L-tryptophan, saccharin and Acyclovir were added to the solution and they were sufficiently mixed together to give a uniform dispersion. Then a 0.1N sodium hydroxide solution was added to the dispersion with stirring till all of the components were completely dissolved. A 0.1N hydrochloric acid solution was added to the resulting solution in small portions to adjust the pH value thereof to 8.0 and distilled water was added thereto to a final volume of the solution of 100 ml.

### Example 23

Polyethylene glycol (Polyethylene Glycol 4000, available from Wako Pure Chemical Industry Co., Ltd.) was dissolved in water, then N-acetyl-L-tryptophan, saccharin and Acyclovir were added to the solution and they were sufficiently mixed together to give a uniform dispersion. Then a 0.1N sodium hydroxide solution was added to the dispersion with stirring till all of the components were completely dissolved. A 0.1N hydrochloric acid solution was added to the resulting solution in small portions to adjust the pH value thereof to 8.0 and distilled water was added thereto to a final volume of the solution of 100 ml.

### Example 24

Sodium chondroitin sulfate (available from Seikagaku Corporation) was dissolved in water, then N-acetyl-L-tryptophan, saccharin and Acyclovir were added to the solution and they were sufficiently mixed together to give a uniform dispersion. Then a 0.1N sodium hydroxide solution was added to the dispersion with stirring till all of the components were completely dissolved. A 0.1N hydrochloric acid solution was added to the resulting solution in small portions to adjust the pH value thereof to 8.0 and distilled water was added thereto to a final volume of the solution of 100 ml.

### Example 25

Sodium hyaluronate (Sodium Hyaluronate HA-Q, available from Q.P. Corporation) was dissolved in hot water, then N-acetyl-L-tryptophan, saccharin and Acyclovir were added to the solution and they were sufficiently mixed together to give a uniform dispersion. Then a 0.1N sodium hydroxide solution was added to the dispersion with stirring till all of the components were completely dissolved. A 0.1N hydrochloric acid solution was added to the resulting solution in small portions to adjust the pH value thereof to 8.0 and distilled water was added thereto to a final volume of the solution of 100 ml.

### Example 26

Sodium alginate (available from Wako Pure Chemical Industry Co., Ltd.) was dissolved in water, then N-acetyl-L-tryptophan, saccharin and Acyclovir were added to the solution and they were sufficiently mixed together to give a uniform dispersion. Then a 0.1N sodium hydroxide solution was added to the dispersion with stirring till all of the components were completely dissolved. A 0.1N hydrochloric acid solution was added to the resulting solution in small portions to adjust the pH value thereof to 8.0 and distilled water was added thereto to a final volume of the solution of 100 ml.

### Example 27

Polyvinyl pyrrolidone was dissolved in warmed water, then N-acetyl-L-tryptophan, saccharin and Acyclovir were added to the solution and they were sufficiently mixed together to give a uniform dispersion. Then a 0.1N sodium hydroxide solution was added to the dispersion with stirring till all of the components were completely dissolved. A 0.1N hydrochloric acid solution was added to the resulting solution in small portions to adjust the pH value thereof to 8.0 and distilled water was added thereto to a final volume of the solution of 100 ml.

### Example 28

Distilled water was added to a mixture of carboxyvinyl polymer (HIVISWAKO 103, available from Wako Pure Chemical Industry Co., Ltd.), N-acetyl-L-tryptophan, saccharin and Acyclovir and they were sufficiently mixed together to give a uniform dispersion. Then a 0.1N sodium hydroxide solution was added to the dispersion with stirring till all of the components were completely dissolved. A 0.1N hydrochloric acid solution was added to the resulting solution in small portions to adjust the pH value thereof to 8.0 and distilled water was added thereto to a final volume of the solution of 100 ml.

### Example 29

Distilled water was added to a mixture of N-acetyl-L-tryptophan, saccharin and Acyclovir and they were mixed together to give a uniform dispersion. Then a 0.1N sodium hydroxide solution was added to the dispersion with stirring till all of these components were completely dissolved. To this solution, there was added a solution of chitosan (Chitosan 10, available from Wako Pure Chemical Industry Co., Ltd.) prepared by adding a 0.1N hydrochloric acid solution till chitosan was completely dissolved, then a 0.1N hydrochloric acid solution was added to the mixture in small portions to thus adjust the pH value thereof to 5.0 and distilled water was added thereto to a final volume of 100 ml.

### Comparative Example 4

Methyl cellulose (Metolose (registered trade mark) SM-15, available from Shin-Etsu Chemical Co., Ltd.) was dispersed in hot water, then the resulting dispersion was cooled, Acyclovir was added thereto at an instance when the dispersion (or solution) became clear and the mixture was sufficiently mixed till a uniform dispersion was obtained. Then a 0.1N sodium hydroxide solution was added to the dispersion with stirring under heating to about 40°C till Acyclovir was completely dissolved. A 0.1N hydrochloric acid solution was gradually added to the solution in small portions to thus adjust the pH value thereof to 8.0 and distilled water was added thereto to a final volume of 100 ml.

### Comparative Example 5

Hydroxypropylmethyl cellulose (Metolose (registered trade mark) 60SH 50, available from Shin-Etsu Chemical Co., Ltd.) was dispersed in hot water and then the resulting dispersion was cooled. Acyclovir was added thereto at an instance when the dispersion (or solution) became clear and the mixture was sufficiently mixed till a uniform dispersion was obtained. Then a 0.1N sodium hydroxide solution was added to the dispersion with stirring under heating to about 40°C till Acyclovir was completely dissolved. A 0.1N hydrochloric acid solution was gradually added to the solution in small portions to thus adjust the pH value thereof to 8.0 and distilled water was added thereto to a final volume of 100 ml.

### Comparative Example 6

Polyvinyl alcohol (Poly(vinyl alcohol) 1000, available from Wako Pure Chemical Industry Co., Ltd.) was dissolved in hot water, then Acyclovir was added to the resulting solution and then they were sufficiently mixed together till a uniform dispersion was obtained. Then a 0.1N sodium hydroxide solution was added to the dispersion with stirring under heating to about 40°C till Acyclovir was completely dissolved. A 0.1N hydrochloric acid solution was gradually added to the solution in small portions to thus adjust the pH value thereof to 8.0 and distilled water was added thereto to a final volume of 100 ml.

### Comparative Example 7

Polyethylene glycol (Polyethylene Glycol 4000 available from Wako Pure Chemical Industry Co., Ltd.) was dissolved in water, then Acyclovir was added to the resulting solution and they were sufficiently mixed together to give a uniform dispersion. Then a 0.1N sodium hydroxide solution was added to the dispersion with stirring under heating to about 40°C till Acyclovir was completely dissolved. A 0.1N hydrochloric acid solution was gradually added to the solution in small portions to thus adjust the pH value thereof to 8.0 and distilled water was added thereto to a final volume of 100 ml.

### Comparative Example 8

Sodium chondroitin sulfate (available from Seikagaku Corporation) was dissolved in water, then Acyclovir was added to the resulting solution and they were sufficiently mixed together to give a uniform dispersion. Then a 0.1N sodium hydroxide solution was added to the dispersion with stirring under heating to about 40°C till Acyclovir was completely dissolved. A 0.1N hydrochloric acid solution was gradually added to the solution in small portions to thus adjust the pH value thereof to 8.0 and distilled water was added thereto to a final volume of 100 ml.

### Comparative Example 9

Sodium hyaluronate (Sodium Hyaluronate HA-Q, available from Q.P. Corporation) was dissolved in hot water, then Acyclovir was added to the resulting solution and they were sufficiently mixed together to give a uniform dispersion. Then a 0.1N sodium hydroxide solution was added to the dispersion with stirring under heating to about 40°C till Acyclovir was completely dissolved. A 0.1N hydrochloric acid solution was gradually added to the solution in small portions to thus adjust the pH value thereof to 8.0 and distilled water was added thereto to a final volume of 100 ml.

### Comparative Example 10

Sodium alginate (available from Wako Pure Chemical Industry Co., Ltd.) was dissolved in water, then Acyclovir was added to the resulting solution and they were sufficiently mixed together to give a uniform dispersion. Then a 0.1N sodium hydroxide solution was added to the dispersion with stirring under heating to about 40°C till Acyclovir was completely dissolved. A 0.1N hydrochloric acid solution was gradually added to the solution in small portions to thus adjust the pH value thereof to 8.0 and distilled water was added thereto to a final volume of 100 ml.

### Comparative Example 11

Polyvinyl pyrrolidone was dissolved in warmed water, then Acyclovir was added to the resulting solution and they were sufficiently mixed together to give a uniform dispersion. Then a 0.1N sodium hydroxide solution was added to the dispersion with stirring under heating to about 40°C till Acyclovir was completely dissolved. A 0.1N hydrochloric acid solution was gradually added to the solution in small portions to thus adjust the pH value thereof to 8.0 and distilled water was added thereto to a final volume of 100 ml.

### Comparative Example 12

Distilled water was added to a mixture of carboxyvinyl polymer (HIVISWAKO 103, available from Wako Pure Chemical Industry Co., Ltd.), while heating to about 40°C and then they were sufficiently mixed to give a uniform dispersion. Then a 0.1N sodium hydroxide solution was added to the dispersion with stirring under heating to about 40°C till Acyclovir was completely dissolved. A 0.1N hydrochloric acid solution was gradually added to the solution in small portions to thus adjust the pH value thereof to 8.0 and distilled water was added thereto to a final volume of 100 ml.

### Comparative Example 13

Distilled water was added to Acyclovir while heating it to about 40°C. Then a 0.1N sodium hydroxide solution was added to the resulting dispersion with stirring till Acyclovir was completely dissolved. To this solution, there was added chitosan (Chitosan 10, available from Wako Pure Chemical Industry Co., Ltd.) dissolved in a 0.1N hydrochloric acid solution. Further a 0.1N hydrochloric acid solution was added thereto in small portions to thus adjust the pH value thereof to 5.0 and distilled water was added thereto to a final volume of 100 ml.

Each of the Acyclovir-containing aqueous pharmaceutical preparations prepared above was charged in a 5 ml volume ampule and then the ampule was sealed through fusion. Then these ampules were stored at 7, 25 or 40°C for about one month to evaluate the stability thereof. The stability was evaluated by determining whether crystals of Acyclovir were separated out from the content of the ampules after the storage or not. The results thus obtained are summarized in the following Table 3.

**Table 3**

| Ex. No. | pH | NAT (%) | SC (%) | WSP (%) | ACV (%) | 7°C | | 25°C | 40°C |
|---|---|---|---|---|---|---|---|---|---|
| | | | | | | 1 week | 4 weeks | 4 weeks | 4 weeks |
| 19 | 8.0 | 5 | 1 | | 0.3 | X | X | ○ | ○ |
| 20 | 8.0 | 5 | 1 | MC SM-15:2 | 0.3 | ○ | ○ | ○ | ○ |
| 21 | 8.0 | 5 | 1 | HPMC 60SH 50: 2 | 0.3 | ○ | ○ | ○ | ○ |
| 22 | 8.0 | 5 | 1 | PVA1000: 2 | 0.3 | ○ | ○ | ○ | ○ |
| 23 | 8.0 | 5 | 1 | PEG4000: 5 | 0.3 | ○ | X | ○ | ○ |
| 24 | 8.0 | 5 | 1 | Na-CS: 2 | 0.3 | ○ | ○ | ○ | ○ |
| 25 | 8.0 | 5 | 1 | Na-HA: 1 | 0.3 | ○ | ○ | ○ | ○ |
| 26 | 8.0 | 5 | 1 | Na-A: 2 | 0.3 | ○ | △ | ○ | ○ |
| 27 | 8.0 | 5 | 1 | PVP-K25: 3 | 0.3 | ○ | ○ | ○ | ○ |
| 28 | 8.0 | 5 | 1 | CVP: 0.01 | 0.3 | ○ | X | ○ | ○ |
| 29 | 5.0 | 5 | 1 | Chitosan: 1 | 0.3 | ○ | ○ | ○ | ○ |
| 4* | 8.0 | -- | -- | MC SM-15: 2 | 0.3 | X | X | X | △ |
| 5* | 8.0 | -- | -- | HPMC 60SH 50: 2 | 0.3 | X | X | X | ○ |
| 6* | 8.0 | -- | -- | PVA1000: 2 | 0.3 | X | X | X | ○ |
| 7* | 8.0 | -- | -- | PEG4000: 5 | 0.3 | X | X | X | △ |
| 8* | 8.0 | -- | -- | Na-CS: 2 | 0.3 | X | X | X | ○ |
| 9* | 8.0 | -- | -- | Na-HA: 1 | 0.3 | X | X | X | ○ |
| 10* | 8.0 | -- | -- | Na-A: 2 | 0.3 | X | X | X | X |
| 11* | 8.0 | -- | -- | PVP-K25: 3 | 0.3 | X | X | X | ○ |
| 12* | 8.0 | -- | -- | CVP: 0.01 | 0.3 | X | X | X | ○ |
| 13* | 5.0 | -- | -- | Chitosan: 1 | 0.3 | X | X | X | △ |

| | | | | | | | | | |
|---|---|---|---|---|---|---|---|---|---|
| *: Comparative Example; | | | | | | | | | |
| MC: methyl cellulose; HPMC: hydroxypropylmethyl cellulose; PVA: polyvinyl alcohol; PEG: polyethylene glycol; PVP: polyvinyl pyrrolidone; WSP: water-soluble polymer; Na-CS: sodium chondroitin sulfate; Na-HA: sodium hyaluronate; Na-A: sodium alginate; CVP: carboxyvinyl polymer. ○ : clear solution; X: separation of crystals; △: fine contaminant | | | | | | | | | |

As will be seen from the results of Comparative Examples in Table 3, any effect of inhibiting crystal-separation is not observed at all for all of the water-soluble polymers examined, when they are used separately. However, if they are used in combination with agents for inhibiting crystal-separation (such as N-acetyl tryptophan, saccharin), the addition of a water-soluble polymer can enhance the effect of inhibiting crystal-separation as compared with the result observed in Example 19 in which only the agent for inhibiting crystal-separation is used, although the degree of the enhancement varies depending on the kinds of the water-soluble polymers.

### Example 30

Methyl cellulose (Metolose (registered trade mark) SM-15 available from Shin-Etsu Chemical Co., Ltd.) was dispersed in hot water and then the resulting dispersion was cooled. Then N-acetyl-DL-tryptophan, saccharin and Acyclovir were added to the dispersion at an instance when the dispersion became clear and they were sufficiently mixed together to give a uniform dispersion. Thereafter, a 0.1N sodium hydroxide solution was added to the dispersion with stirring till all of the components were completely dissolved. To the resulting solution, there were added boric acid, disodium ethylenediaminetetraacetate (Sodium EDTA) and benzalkonium chloride, then a 0.1N hydrochloric acid solution was gradually added to the solution to adjust the pH value thereof to 8.0 and distilled water was added there to give a solution having a final volume of 100 ml.

### Example 31

Methyl cellulose (Metolose (registered trade mark) SM-15 available from Shin-Etsu Chemical Co., Ltd.) was dispersed in hot water and then the resulting dispersion was cooled. Then N-acetyl-DL-tryptophan, saccharin and Acyclovir were added to the dispersion at an instance when the dispersion became clear and they were sufficiently mixed together to give a uniform dispersion. Thereafter, a 0.1N sodium hydroxide solution was added to the dispersion with stirring till all of the components were completely dissolved. To the resulting solution, there were added boric acid and a solution of p-hydroxybenzoic acid methyl ester and p-hydroxybenzoic acid propyl ester in propylene glycol (1 in 100 solution) prepared in advance, then a 0.1N hydrochloric acid solution was gradually added to the solution to adjust the pH value thereof to 5.5 and distilled water was added there to give a solution having a final volume of 100 ml.

### Example 32

Methyl cellulose (Metolose (registered trade mark) SM-15 available from Shin-Etsu Chemical Co., Ltd.) was dispersed in hot water and then the resulting dispersion was cooled. Then N-acetyl-DL-tryptophan, saccharin and Acyclovir were added to the dispersion at an instance when the dispersion became clear and they were sufficiently mixed together to give a uniform dispersion. Thereafter, a 0.1N sodium hydroxide solution was added to the dispersion with stirring till all of the components were completely dissolved. To the resulting solution, there were added boric acid and chlorobutanol, then a 0.1N hydrochloric acid solution was gradually added to the solution to adjust the pH value thereof to 5.5. Then distilled water was added there to give a solution having a final volume of 100 ml.

### Example 33

Methyl cellulose (Metolose (registered trade mark) SM-15 available from Shin-Etsu Chemical Co., Ltd.) was dispersed in hot water and then the resulting dispersion was cooled. Then N-acetyl-DL-tryptophan, saccharin and Acyclovir were added to the dispersion at an instance when the dispersion became clear and they were sufficiently mixed together to give a uniform dispersion. Thereafter, a 0.1N sodium hydroxide solution was added to the dispersion with stirring till all of the components were completely dissolved. To the resulting solution, there was added chlorobutanol, then a 0.1N hydrochloric acid solution was gradually added to the solution to adjust the pH value thereof to 5.5 and distilled water was added there to give a solution having a final volume of 100 ml.

Each of the Acyclovir-containing aqueous pharmaceutical preparations prepared above was introduced into a 5 ml volume ampule and then the ampule was sealed through fusion. Then these ampules were stored at 7, 25 and 40°C over about one month to evaluate the stability thereof. The stability was evaluated by determining whether crystals of Acyclovir were separated out from the content of the ampules after the storage thereof or not. The results thus obtained are listed in the following Table 4.

**Table 4**

| Ex. No. | | 30 | 31 | 32 | 33 |
|---|---|---|---|---|---|
| Acyclovir | | 0.3 | 0.3 | 0.3 | 0.5 |
| N-acetyl tryptophan | | 3.0 | 3.0 | 3.0 | 5.8 |
| Saccharin | | 0.6 | 0.6 | 0.6 | 1.2 |
| Methyl cellulose | | 0.5 | 0.5 | 0.5 | 0.5 |
| Boric acid | | 0.1 | 0.1 | 0.1 | -- |
| Sodium EDTA | | 0.005 | -- | -- | -- |
| Benzalkonium chloride | | 0.005 | -- | -- | -- |
| Propylene glycol | | -- | 1.0 | -- | -- |
| Methylparaben | | -- | 0.026 | -- | -- |
| Propylparaben | | -- | 0.014 | -- | -- |
| Chlorobutanol | | -- | -- | 0.5 | 0.5 |
| pH | | 8.0 | 5.5 | 5.5 | 5.5 |
| 7°C; | 4 weeks | ○ | ○ | ○ | ○ |
| 25°C; | 4 weeks | ○ | ○ | ○ | ○ |
| 40°C; | 4 weeks | ○ | ○ | ○ | ○ |
| Note: Numerical values appearing in Table 4 are expressed in terms of "W/V%". ○ : Clear solution; X : Separation of crystals; △ : Fine contaminant. | | | | | |

There was not any crystal-separation at all the temperatures examined even in formulations, which comprise a typical preservative such as benzalkonium chloride, paraben or chlorobutanol, as will be seen from the results observed in Examples 30 to 32 in Table 4 and these formulations show excellent storage stability. Moreover, the results observed in Example 33 indicate that the formulation, which comprises chlorobutanol and Acyclovir at a higher concentration never undergoes any crystal-separation and shows excellent storage stability. All of the formulations prepared in Examples 30 to 33 have a sufficient preservative efficacy.

### Example 34

Methyl cellulose (Metolose (registered trade mark) SM-15 available from Shin-Etsu Chemical Co., Ltd.) (2 g) was dispersed in hot water and then the resulting dispersion was cooled. Then 10 g of N-acetyl-L-tryptophan, 5 g of saccharin and 0.9 g of Gancyclovir were added to the dispersion at an instance when the dispersion became clear and they were sufficiently mixed together to give a uniform dispersion. Thereafter, a 0.1N sodium hydroxide solution was added to the dispersion with stirring till all of the components were completely dissolved. Then a 0.1N hydrochloric acid solution was gradually added to the resulting solution in small portions to adjust the pH value thereof to 8.0 and distilled water was added there to give a solution having a final volume of 100 ml.

The Gancyclovir aqueous solution thus prepared was introduced into a 5 ml volume ampule and then the ampule was sealed through fusion. Then the sample was stored at 7, 25 and 40°C over one month to evaluate the stability thereof. The stability was evaluated by determining whether crystals of Gancyclovir were separated out from the content of the sample after the storage thereof or not. The results thus obtained are listed in the following Table 5.

It was thus confirmed that even if Gancyclovir was substituted for Acyclovir as a drug, the aqueous pharmaceutical preparation according to the present invention never underwent any crystal-separation and could stably be stored.

| | |
|---|---|
| Gancyclovir: | 0.9 g |
| N-Acetyl-L-tryptophan: | 10 g |
| Saccharin: | 5 g |
| Methyl cellulose: | 2 g |
| 0.1N Sodium hydroxide aqueous solution: | sufficient quantity (q.s.) |
| 0.1N Hydrochloric acid aqueous solution: | sufficient quantity (q.s.) |
| Distilled water: | sufficient quantity (q.s.) |
| Total | 100 ml |

**Table 5**

| Conditions for Storage | Quality |
|---|---|
| Immediately After Preparation | Clear Solution |
| 7°C, one month | No change |
| 25°C, one month | No change |
| 40°C, one month | No change |

### Example 35

Distilled water was added to a mixture of Vidarabine, N-acetyl-L-tryptophan and saccharin and they were sufficiently mixed till a uniform dispersion was obtained. Then 0.1N sodium hydroxide solution was added to the dispersion with stirring till all of the components were completely dissolved. A 0.1N hydrochloric acid solution was added to the resulting solution in small portions to adjust the pH value thereof to 8.0 and distilled water was added thereto to a final volume of 100 ml.

### Example 36

Distilled water was added to a mixture of Idoxuridine, N-acetyl-L-tryptophan and saccharin and they were sufficiently mixed till a uniform dispersion was obtained. Then 0.1N sodium hydroxide solution was added to the dispersion with stirring till all of the components were completely dissolved. A 0.1N hydrochloric acid solution was added to the resulting solution in small portions to adjust the pH value thereof to 8.0 and distilled water was added thereto to a final volume of 100 ml.

### Example 37

Distilled water was added to a mixture of Trifluorothymidine, N-acetyl-L-tryptophan and saccharin and they were sufficiently mixed till a uniform dispersion was obtained. Then 0.1N sodium hydroxide solution was added to the dispersion with stirring till all of the components were completely dissolved. A 0.1N hydrochloric acid solution was added to the resulting solution in small portions to adjust the pH value thereof to 8.0 and distilled water was added thereto to a final volume of 100 ml.

### Example 38

Distilled water was added to a mixture of cytosine arabinoside, N-acetyl-L-tryptophan and saccharin and they were sufficiently mixed till a uniform dispersion was obtained. Then 0.1N sodium hydroxide solution was added to the dispersion with stirring till all of the components were completely dissolved. A 0.1N hydrochloric acid solution was added to the resulting solution in small portions to adjust the pH value thereof to 8.0 and distilled water was added thereto to a final volume of 100 ml.

### Example 39

Distilled water was added to a mixture of Bromodeoxyuridine, N-acetyl-L-tryptophan and saccharin and they were sufficiently mixed till a uniform dispersion was obtained. Then 0.1N sodium hydroxide solution was added to the dispersion with stirring till all of the components were completely dissolved. A 0.1N hydrochloric acid solution was added to the resulting solution in small portions to adjust the pH value thereof to 8.0 and distilled water was added thereto to a final volume of 100 ml.

### Example 40

Distilled water was added to a mixture of 5-iodo-2-deoxycytidine, N-acetyl-L-tryptophan and saccharin and they were sufficiently mixed till a uniform dispersion was obtained. Then 0.1N sodium hydroxide solution was added to the dispersion with stirring till all of the components were completely dissolved. A 0.1N hydrochloric acid solution was added to the resulting solution in small portions to adjust the pH value thereof to 8.0 and distilled water was added thereto to a final volume of 100 ml.

### Comparative Example 14

Distilled water was added to Vidarabine and they were sufficiently mixed to give a uniform dispersion. Then a 0.1N sodium hydroxide solution was added to the resulting dispersion with stirring till all of the components were completely dissolved. A 0.1N hydrochloric acid solution was added to the resulting solution in small portions to adjust the pH value thereof to 8.0 and distilled water was added thereto to a final volume of 100 ml.

### Comparative Example 15

Distilled water was added to Idoxuridine and they were sufficiently mixed to give a uniform dispersion. Then a 0.1N sodium hydroxide solution was added to the resulting dispersion with stirring till all of the components were completely dissolved. A 0.1N hydrochloric acid solution was added to the resulting solution in small portions to adjust the pH value thereof to 8.0 and distilled water was added thereto to a final volume of 100 ml.

### Comparative Example 16

Distilled water was added to Trifluorothymidine and they were sufficiently mixed to give a uniform dispersion. Then a 0.1N sodium hydroxide solution was added to the resulting dispersion with stirring till all of the components were completely dissolved. A 0.1N hydrochloric acid solution was added to the resulting solution in small portions to adjust the pH value thereof to 8.0 and distilled water was added thereto to a final volume of 100 ml.

### Comparative Example 17

Distilled water was added to cytosine arabinoside and they were sufficiently mixed to give a uniform dispersion. Then a 0.1N sodium hydroxide solution was added to the resulting dispersion with stirring till all of the components were completely dissolved. A 0.1N hydrochloric acid solution was added to the resulting solution in small portions to adjust the pH value thereof to 8.0 and distilled water was added thereto to a final volume of 100 ml.

### Comparative Example 18

Distilled water was added to Bromodeoxyuridine and they were sufficiently mixed to give a uniform dispersion. Then a 0.1N sodium hydroxide solution was added to the resulting dispersion with stirring till all of the components were completely dissolved. A 0.1N hydrochloric acid solution was added to the resulting solution in small portions to adjust the pH value thereof to 8.0 and distilled water was added thereto to a final volume of 100 ml.

### Comparative Example 19

Distilled water was added to 5-iodo-2-deoxycytidine and they were sufficiently mixed to give a uniform dispersion. Then a 0.1N sodium hydroxide solution was added to the resulting dispersion with stirring till all of the components were completely dissolved. A 0.1N hydrochloric acid solution was added to the resulting solution in small portions to adjust the pH value thereof to 8.0 and distilled water was added thereto to a final volume of 100 ml.

Each of the drug-containing aqueous pharmaceutical preparations prepared above was introduced into a 5 ml volume ampule and then the ampule was sealed through fusion. Then these ampules were stored at 7°C over 14 days to evaluate the stability thereof. The stability was evaluated by determining whether crystals of Acyclovir were separated out from the content of the ampules after the storage thereof or not. The results thus obtained are listed in the following Table 6.

**Table 6**

| Ex. No. | pH | NAT (%) | SC (%) | Antiviral Agent | | 7°C | | |
|---|---|---|---|---|---|---|---|---|
| | | | | | | Day | | |
| | | | | Name of Drug | C(%) | 3 | 7 | 14 |
| 35 | 8.0 | 5 | 1 | Vidarabine | 0.1 | ○ | ○ | ○ |
| 36 | 8.0 | 5 | 1 | Idoxuridine | 0.3 | ○ | ○ | ○ |
| 37 | 8.0 | 5 | 1 | Trifluorothymidine | 5.0 | ○ | ○ | ○ |
| 38 | 8.0 | 5 | 1 | Cytosine arabinoside | 5.0 | ○ | ○ | ○ |
| 39 | 8.0 | 5 | 1 | Bromodeoxyuridine | 5.0 | ○ | ○ | X |
| 40 | 8.0 | 5 | 1 | 5-Iodo-2-deoxycytidine | 2.5 | ○ | X | X |
| 14* | 8.0 | -- | -- | Vidarabine | 0.1 | X | X | X |
| 15* | 8.0 | -- | -- | Idoxuridine | 0.3 | X | X | X |
| 16* | 8.0 | -- | -- | Trifluorothymidine | 5.0 | ○ | ○ | X |
| 17* | 8.0 | -- | -- | Cytosine arabinoside | 5.0 | ○ | ○ | X |
| 18* | 8.0 | -- | -- | Bromodeoxyuridine | 5.0 | X | X | X |
| 19* | 8.0 | -- | -- | 5-Iodo-2-deoxycytidine | 2.5 | X | X | X |

| | | | | | | | | |
|---|---|---|---|---|---|---|---|---|
| *: Comparative Example; | | | | | | | | |
| C: concentration; NAT: N-acetyl tryptophan; SC: saccharin. ○ : clear solution; X: crystal-separation; △: fine contaminant. | | | | | | | | |

The results listed in Table 6 dearly indicate that all of the drugs used in Examples 35 to 40 permit the inhibition of crystal-separation and the improvement of storage stability, as compared with the formulations prepared in Comparative Examples 14 to 19, which are free of any agent for inhibiting crystal-separation.

It is clear from the foregoing that the aqueous pharmaceutical preparation permits the inhibition of crystal-separation and can stably be stored, even if the pharmaceutical preparation comprises an antiviral agent having a purine skeleton other than Acyclovir and Gancyclovir as well as an antiviral agent having a pyrimidine skeleton such as Idoxuridine.

### Example of Formulation 1

To hot water, there was dispersed 5 g of methyl cellulose (Metolose (registered trade mark) SM-15 available from Shin-Etsu Chemical Co., Ltd.), followed by cooling the resulting dispersion. Then 58 g of N-acetyl tryptophan, 12 g of saccharin and 5 g of Acyclovir were added to the dispersion at an instance when the dispersion became clear and they were sufficiently mixed to give a uniform dispersion. Subsequently, a 0.1N sodium hydroxide solution was added to the dispersion with stirring till all of the components were completely dissolved. To the resulting solution, there were added 5 g of ε -aminocaproic acid and 5 g of chlorobutanol. Then a 0.1N hydrochloric acid solution was gradually added to the mixture in small portions to thus adjust the pH value thereof to 5.5 and distilled water was added to the solution to a final volume of 1000 ml. The resulting solution was sterilized by passing through a membrane filter having a pore size of 0.22 µm and then charged in eye drop bottles to give a 0.5% Acyclovir eye drop solution.

| | |
|---|---|
| Acyclovir | 5 g |
| N-Acetyl tryptophan | 58 g |
| Saccharin | 12 g |
| Methyl cellulose | 5 g |
| ε-Aminocaproic acid | 5 g |
| Chlorobutanol | 5 g |
| 0.1N NaOH aqueous solution | Sufficient quantity |
| 0.1N HCl aqueous solution | Sufficient quantity |
| Distilled water | Sufficient quantity |
| Total | 1000 ml |

### Example of Formulation 2

To hot water, there was added 5 g of methyl cellulose (Metolose (registered trade mark) SM-15 available from Shin-Etsu Chemical Co., Ltd.), followed by cooling the resulting dispersion. Then 30 g of N-acetyl tryptophan, 6 g of saccharin and 3 g of Acyclovir were added to the dispersion at an instance when the dispersion became clear and they were sufficiently mixed to give a uniform dispersion. Subsequently, a 0.1N sodium hydroxide solution was added to the dispersion with stirring till all of the components were completely dissolved. To the resulting solution, there were added 1 g of boric acid, 0.26 g of p-hydroxybenzoic acid methyl ester and 0.14 g of p-hydroxybenzoic acid propyl ester. Then a 0.1N hydrochloric acid solution was gradually added to the mixture in small portions to thus adjust the pH value thereof to 5.5 and distilled water was added to the solution to a final volume of 1000 ml. The resulting solution was sterilized by passing through a membrane filter having a pore size of 0.22µm and then charged in eye drop bottles to give a 0.3% Acyclovir eye drop solution.

| | |
|---|---|
| Acyclovir | 3 g |
| N-Acetyl tryptophan | 30 g |
| Saccharin | 6 g |
| Methyl cellulose | 5 g |
| Boric acid | 1 g |
| p-Hydroxybenzoic acid methyl ester | 0.26 g |
| p-Hydroxybenzoic acid propyl ester | 0.14 g |
| 0.1N NaOH aqueous solution | Sufficient quantity |
| 0.1N HCl aqueous solution | Sufficient quantity |
| Distilled water | Sufficient quantity |
| Total | 1000 ml |

### Example of Formulation 3

To hot water, there was dispersed 5 g of methyl cellulose (Metolose (registered trade mark) SM-15 available from Shin-Etsu Chemical Co., Ltd.), followed by cooling the resulting dispersion. Then 50 g of N-acetyl tryptophan, 50 g of saccharin and 7 g of Acyclovir were added to the dispersion at an instance when the dispersion became clear and they were sufficiently mixed to give a uniform dispersion. Subsequently, a 0.1N sodium hydroxide solution was added to the dispersion with stirring till all of the components were completely dissolved. To the resulting solution, there were added 1 g of boric acid, 0.06 g of disodium ethylenediaminetetraacetate (Sodium EDTA) and 0.05 g of benzalkonium chloride. Then a 0.1N hydrochloric acid solution was gradually added to the mixture in small portions to thus adjust the pH value thereof to 8.0 and distilled water was added to the solution to a final volume of 1000 ml. The resulting solution was sterilized by passing through a membrane filter having a pore size of 0.22µm and then charged in eye drop bottles to give a 0.7% Acyclovir eye drop solution.

| | |
|---|---|
| Acyclovir | 7 g |
| N-Acetyl tryptophan | 50 g |
| Saccharin | 50 g |
| Methyl cellulose | 5 g |
| Boric acid | 1 g |
| Sodium EDTA | 0.06 g |
| Benzalkonium chloride | 0.05 g |
| 0.1N NaOH aqueous solution | Sufficient quantity |
| 0.1N HCl aqueous solution | Sufficient quantity |
| Distilled water | Sufficient quantity |
| Total | 1000 ml |

### Example of Formulation 4

To hot water, there was dispersed 20 g of methyl cellulose (Metolose (registered trade mark) SM-15 available from Shin-Etsu Chemical Co., Ltd.), followed by cooling the resulting dispersion. Then 58 g of N-acetyl tryptophan, 12 g of saccharin and 5 g of Acyclovir were added to the dispersion at an instance when the dispersion became clear and they were sufficiently mixed to give a uniform dispersion. Subsequently, a 0.1N sodium hydroxide solution was added to the dispersion with stirring till all of the components were completely dissolved. To the resulting solution, there were added 1 g of boric acid, 0.26 g of p-hydroxybenzoic acid methyl ester and 0.14 g of p-hydroxybenzoic acid propyl ester. Then a 0.1N hydrochloric acid solution was gradually added to the mixture in small portions to thus adjust the pH value thereof to 5.5 and distilled water was added to the solution to a final volume of 1000 ml. The resulting solution was sterilized by passing through a membrane filter having a pore size of 0.22µm and then charged in nasal quantitative atomizers to give a 0.5% Acyclovir nasal drop solution.

| | |
|---|---|
| Acyclovir | 5 g |
| N-Acetyl tryptophan | 58 g |
| Saccharin | 12 g |
| Methyl cellulose | 20 g |
| Boric acid | 1 g |
| P-Hydroxybenzoic acid methyl ester | 0.26 g |
| p-Hydxoxybenzoic acid propyl ester | 0.14 g |
| 0.1N NaOH aqueous solution | Sufficient quantity |
| 0.1N HCl aqueous solution | Sufficient quantity |
| Distilled water | Sufficient quantity |
| Total | 1000 ml |

### Example of Formulation 5

To hot water, there was dispersed 20 g of methyl cellulose (Metolose (registered trade mark) SM-15 available from Shin-Etsu Chemical Co., Ltd.), followed by cooling the resulting dispersion. Then 50 g of N-acetyl tryptophan, 10 g of saccharin and 3 g of Acyclovir were added to the dispersion at an instance when the dispersion became clear and they were sufficiently mixed to give a uniform dispersion. Subsequently, a 0.1N sodium hydroxide solution was added to the dispersion with stirring till all of the components were completely dissolved. To the resulting solution, there were added 1 g of boric acid, 0.06 g of disodium ethylenediaminetetraacetate (Sodium EDTA) and 0.05 g of benzalkonium chloride. Then a 0.1N hydrochloric acid solution was gradually added to the mixture in small portions to thus adjust the pH value thereof to 8.0 and distilled water was added to the solution to a final volume of 1000 ml. The resulting solution was sterilized by passing though a membrane filter having a pore size of 0.22µm and then charged in dropping bottles to give a 0.3% Acyclovir ear drop solution.

| | |
|---|---|
| Acyclovir | 3 g |
| N-Acetyl tryptophan | 50 g |
| Saccharin | 10 g |
| Methyl cellulose | 20 g |
| Boric acid | 1 g |
| Sodium EDTA | 0.06 g |
| Benzalkonium chloride | 0.05 g |
| 0.1N NaOH aqueous solution | Sufficient quantity |
| 0.1N HCl aqueous solution | Sufficient quantity |
| Distilled water | Sufficient quantity |
| Total | 1000 ml |

### Example of Formulation 6

To hot water, there was dispersed 20 g of methyl cellulose (Metolose (registered trade mark) SM-15 available from Shin-Etsu Chemical Co., Ltd.), followed by cooling the resulting dispersion. Then 50 g of N-acetyl tryptophan, 50 g of saccharin and 5 g of Acyclovir were added to the dispersion at an instance when the dispersion became clear and they were sufficiently mixed to give a uniform dispersion. Subsequently, a 0.1N sodium hydroxide solution was added to the dispersion with stirring till all of the components were completely dissolved. To the resulting solution, there were added 2 g of sodium citrate, 0.26 g of p-hydroxybenzoic acid methyl ester and 0.14 g of p-hydroxybenzoic add propyl ester. Then a 0.1N hydrochloric acid solution was gradually added to the mixture in small portions to thus adjust the pH value thereof to 5.5 and distilled water was added to the solution to a final volume of 1000 ml. The resulting solution was sterilized by passing through a membrane filter having a pore size of 0.22µm and then charged in electric nebulizers to give a 0.5% Acyclovir inhalant.

| | |
|---|---|
| Acyclovir | 5 g |
| N-Acetyl tryptophan | 50 g |
| Saccharin | 50 g |
| Methyl cellulose | 20 g |
| Sodium citrate | 2 g |
| P-Hydroxybenzoic acid methyl ester | 0.26 g |
| p-Hydroxybenzoic acid propyl ester | 0.14 g |
| 0.1N NaOH aqueous solution | Sufficient quantity |
| 0.1N HCl aqueous solution | Sufficient quantity |
| Distilled water | Sufficient quantity |
| Total | 1000 ml |

### Example of Formulation 7

To hot water, there was dispersed 20 g of methyl cellulose (Metolose (registered trade mark) SM-15 available from Shin-Etsu Chemical Co., Ltd.), followed by cooling the resulting dispersion. Then 100 g of N-acetyl tryptophan, 50 g of saccharin and 9 g of Acyclovir were added to the dispersion at an instance when the dispersion became clear and they were sufficiently mixed to give a uniform dispersion. Subsequently, a 0.1N sodium hydroxide solution was added to the dispersion with stirring till all of the components were completely dissolved. To the resulting solution, there were added 2 g of sodium citrate, 0.26 g of p-hydroxybenzoic acid methyl ester and 0.14 g of p-hydroxybenzoic acid propyl ester. Then a 0.1N hydrochloric acid solution was gradually added to the mixture in small portions to thus adjust the pH value thereof to 5.5 and distilled water was added to the solution to a final volume of 1000 ml. The resulting solution was sterilized by passing through a membrane filter having a pore size of 0.22µm and then charged in atomizers to give a 0.9% Acyclovir aerosol.

| | |
|---|---|
| Acyclovir | 9 g |
| N-Acetyl tryptophan | 100 g |
| Saccharin | 50 g |
| Methyl cellulose | 20 g |
| Sodium citrate | 4.4 g |
| p-Hydroxybenzoic acid methyl ester | 0.26 g |
| p-Hydroxybenzoic acid propyl ester | 0.14 g |
| 0.1N NaOH aqueous solution | Sufficient quantity |
| 0.1N HCl aqueous solution | Sufficient quantity |
| Distilled water | Sufficient quantity |
| Total | 1000 ml |

### Example of Formulation 8

To hot water, there was dispersed 20 g of methyl cellulose (Metolose (registered trade mark) SM-15 available from Shin-Etsu Chemical Co., Ltd.), followed by cooling the resulting dispersion. Then 100 g of saccharin and 3 g of Acyclovir were added to the dispersion at an instance when the dispersion became clear and they were sufficiently mixed to give a uniform dispersion. Subsequently, a 0.1N sodium hydroxide solution was added to the dispersion with stirring till all of the components were completely dissolved. To the resulting solution, there were added 2 g of sodium citrate, 0.1 ml of a 5% p-hydroxybenzoic acid ethyl ester prepared in advance (36 v/v% ethanol solution). Then a 0.1N hydrochloric acid solution was gradually added to the mixture in small portions to thus adjust the pH value thereof to 5.5 and distilled water was added to the solution to a final volume of 1000 ml. The resulting solution was sterilized by passing through a membrane filter having a pore size of 0.22µm and then charged in ampules and then sealed through fusion to give a 0.3% Acyclovir pharmaceutical preparation for internal use.

| | |
|---|---|
| Acyclovir | 3 g |
| Saccharin | 100 g |
| Methyl cellulose | 20 g |
| Sodium citrate | 2 g |
| p-Hydroxybenzoic acid ethyl ester | 0.5 g |
| 0.1N NaOH aqueous solution | Sufficient quantity |
| 0.1N HCl aqueous solution | Sufficient quantity |
| Distilled water | Sufficient quantity |
| Total | 1000 ml |

### Example of Formulation 9

To hot water, there was dispersed 20 g of methyl cellulose (Metolose SM-15 available from Shin-Etsu Chemical Co., Ltd.) and the resulting dispersion was cooled. Then 100 g of N-acetyl tryptophan and 5 g of Acyclovir were added to the dispersion at an instance when the dispersion became clear and the resulting mixture was sufficiently mixed to give a uniform dispersion. Subsequently, a 0.1N sodium hydroxide solution was added to the dispersion with stirring till all of the components were completely dissolved. To the resulting solution, there was added 5 g of sodium citrate. Then a 0.1N hydrochloric acid solution was added thereto in small portions to thus adjust the pH value of the solution to 8.0 and distilled water was added thereto to a final volume of 1000 ml. The resulting solution was sterilized by passing through a membrane filter having a pore size of 0.22µm, then charged in ampules and sealed through fusion to give a 0.5% Acyclovir injection.

| | |
|---|---|
| Acyclovir | 5 g |
| N-Acetyl tryptophan | 100 g |
| Methyl cellulose | 20 g |
| Sodium citrate | 5 g |
| 0.1N NaOH aqueous solution | Sufficient quantity |
| 0.1N HCl aqueous solution | Sufficient quantity |
| Distilled water | Sufficient quantity |
| Total | 1000 ml |

### Example of Formulation 10

To hot water, there was dispersed 20 g of methyl cellulose (Metolose SM-15 available from Shin-Etsu Chemical Co., Ltd.) and the resulting dispersion was cooled. Then 50 g of N-acetyl tryptophan, 50 g of saccharin and 5 g of Acyclovir were added to the dispersion at an instance when the dispersion became clear and the resulting mixture was sufficiently mixed to give a uniform dispersion. Subsequently, a 0.1N sodium hydroxide solution was added to the dispersion with stirring till all of the components were completely dissolved. To the resulting solution, there was added 5 g of sodium citrate. Then a 0.1N hydrochloric acid solution was added thereto in small portions to thus adjust the pH value of the solution to 8.0 and distilled water was added thereto to a final volume of 1000 ml. The resulting solution was sterilized by passing through a membrane filter having a pore size of 0.22µm, then charged in ampules and sealed through fusion to give a 0.5% Acyclovir injection.

| | |
|---|---|
| Acyclovir | 5 g |
| N-Acetyl tryptophan | 50 g |
| Saccharin | 50 g |
| Methyl cellulose | 20 g |
| Sodium citrate | 5 g |
| 0.1N NaOH aqueous solution | Sufficient quantity |
| 0.1N HCl aqueous solution | Sufficient quantity |
| Distilled water | Sufficient quantity |
| Total | 1000 ml |

### Example of Formulation 11

To hot water, there was dispersed 20 g of methyl cellulose (Metolose SM-15 available from Shin-Etsu Chemical Co., Ltd.) and the resulting dispersion was cooled. Then 100 g of N-acetyl tryptophan and 5 g of Gancyclovir were added to the dispersion at an instance when the dispersion became clear and the resulting mixture was sufficiently mixed to give a uniform dispersion. Subsequently, a 0.1N sodium hydroxide solution was added to the dispersion with stirring till all of the components were completely dissolved. To the resulting solution, there was added 2.5 g of sodium citrate. Then a 0.1N hydrochloric acid solution was added thereto in small portions to thus adjust the pH value of the solution to 8.0 and distilled water was added thereto to a final volume of 1000 ml. The resulting solution was sterilized by passing through a membrane filter having a pore size of 0.22µm, then charged in ampules and sealed through fusion to give a 0.5% Gancyclovir intraocular injection.

| | |
|---|---|
| Gancyclovir | 5 g |
| N-Acetyl tryptophan | 100 g |
| Methyl cellulose | 20 g |
| Sodium citrate | 2.5 g |
| 0.1N NaOH aqueous solution | Sufficient quantity |
| 0.1N HCl aqueous solution | Sufficient quantity |
| Distilled water | Sufficient quantity |
| Total | 1000 ml |

### Example of Formulation 12

To hot water, there was dispersed 20 g of methyl cellulose (Metolose (registered trade mark) SM-15 available from Shin-Etsu Chemical Co., Ltd.) and the resulting dispersion was cooled. Then 50 g of N-acetyl tryptophan, 10 g of saccharin and 3 g of Idoxuridine were added to the dispersion at an instance when the dispersion became clear and they were sufficiently mixed together to give a uniform dispersion. Then a 0.1N sodium hydroxide solution was added to the dispersion with stirring till all of the components were completely dissolved. To the resulting solution, there were added 1 g of boric acid, 0.06 g of disodium ethylenediaminetetra-acetate (Sodium EDTA) and 0.05 g of benzalkonium chloride. A 0.1N hydrochloric acid solution was then added to the resulting mixture in small portions to adjust the pH value thereof to 8.0 and distilled water was added thereto to a final volume of 1000 ml. The resulting solution was sterilized by passing it through a membrane filter having a pore size of 0.22µm and charged in eye drop bottles to thus give a 0.3% Idoxuridine eye drop solution.

| | |
|---|---|
| Idoxuridine | 3 g |
| N-Acetyl tryptophan | 50 g |
| Saccharin | 10 g |
| Methyl cellulose | 20 g |
| Boric acid | 1 g |
| Sodium EDTA | 0.06 g |
| Benzalkonium chloride | 0.05 g |
| 0.1N NaOH aqueous solution | Sufficient quantity |
| 0.1N HCl aqueous solution | Sufficient quantity |
| Distilled water | Sufficient quantity |
| Total | 1000 ml |

### Example of Formulation 13

To hot water, there was dispersed 20 g of methyl cellulose (Metolose (registered trade mark) SM-15 available from Shin-Etsu Chemical Co., Ltd.) and the resulting dispersion was cooled. Then 50 g of N-acetyl tryptophan, 10 g of saccharin and 25 g of Trifluorothymidine were added to the dispersion at an instance when the dispersion became clear and they were sufficiently mixed together to give a uniform dispersion. Subsequently, a 0.1N sodium hydroxide solution was added to the dispersion with stirring till all of the components were completely dissolved. To the resulting solution, there were added 1 g of boric acid, 0.06 g of disodium ethylenediaminetetraacetate (Sodium EDTA) and 0.05 g of benzalkonium chloride. A 0.1N hydrochloric acid solution was added to the resulting solution in small portions to adjust the pH value of the solution to 8.0 and distilled water was added thereto to a final volume of 1000 ml. The resulting solution was sterilized by passing it through a membrane filter having a pore size of 0.22 µm, then charged in eye drop bottles to give a 2.5% Trifluorothymidine eye drop solution.

| | |
|---|---|
| Trifluorothymidine | 25 g |
| N-Acetyl tryptophan | 50 g |
| Saccharin | 10 g |
| Methyl cellulose | 20 g |
| Boric acid | 1 g |
| Sodium EDTA | 0.06 g |
| Benzalkonium chloride | 0.05 g |
| 0.1N NaOH aqueous solution | Sufficient quantity |
| 0.1N HCl aqueous solution | Sufficient quantity |
| Distilled water | Sufficient quantity |
| Total | 1000 ml |

### Industrial Applicability

The present invention permits the preparation of an aqueous pharmaceutical preparation, which comprises, as an effective component, an antiviral agent having a purine skeleton or a pyrimidine skeleton and represented by Acyclovir or Idoxuridine, at a pH value near the physiologically acceptable region. The aqueous pharmaceutical preparation according to the present invention comprises, in addition to an antiviral agent, N-acetyl tryptophan and/or saccharin and pharmaceutically acceptable salts thereof as an agent for inhibiting crystal-separation as well as a water-soluble polymer as an optional auxiliary agent for inhibiting crystal-separation and can be prepared and stored at a pH value near the physiologically acceptable region.

In the aqueous pharmaceutical preparation of the present invention, an antiviral agent having a purine skeleton or a pyrimidine skeleton is dissolved in water within the physiologically acceptable pH region and any separation of crystals thereof during the storage is inhibited.

The aqueous pharmaceutical preparation of the present invention can be used as a remedy for treating, for instance, infectious diseases attributable to herpesviruses such as chickenpox, herpes encephalitis-meningitis and herpes simplex; infectious diseases attributable to cytomegaloviruses; and acquired immunodeficiency syndrome, in the form of, for instance, an eye drop, a nasal drop, an ear drop, an inhalant, an aerosol, a liquid preparation for internal use and an injection.

## Claims

1. An aqueous pharmaceutical preparation comprising an antiviral agent having a purine skeleton or a pyrimidine skeleton or a pharmaceutically acceptable salt thereof; an agent for inhibiting crystal-separation and water.

2. The aqueous pharmaceutical preparation of claim 1 wherein the agent for inhibiting crystal-separation comprises at least one member selected from the group consisting of acetyl tryptophan, saccharin and pharmaceutically acceptable salts thereof.

3. The aqueous pharmaceutical preparation of claim 1 or 2 wherein it further comprises a water-soluble polymer as an auxiliary agent for inhibiting crystal-separation.

4. The aqueous pharmaceutical preparation of claim 3 wherein the water-soluble polymer is methyl cellulose and/or hydroxypropylmethyl cellulose.

5. The aqueous pharmaceutical preparation of claim 1 wherein the antiviral agent comprises at least one member selected from the group consisting of Acyclovir, Gancyclovir, Vidarabine, Idoxuridine, Trifluorothymidine, cytosine arabinoside, Bromodeoxyuridine and 5-iodo-2-deoxycytidine.

6. The aqueous pharmaceutical preparation as set forth in any one of claims 1 to 5 wherein the pH value ranges from 3 to 10.

7. The aqueous pharmaceutical preparation as set forth in any one of claims 1 to 6 wherein it further comprises at least one member selected from the group consisting of pharmaceutically acceptable buffering agents, isotonicities, preservatives and stabilizers.

8. The aqueous pharmaceutical preparation as set forth in any one of claims 1 to 7 wherein the aqueous pharmaceutical preparation is an eye drop, a nasal drop, an ear drop, an inhalant, an aerosol, a liquid preparation for internal use or an injection.
